# FASCICULE DE BREVET EUROPEEN

(11) **EP 2 127 614 B1**
(45) Date de publication et mention de la délivrance du brevet: **16.02.2011**
(21) Numéro de dépôt: 09161130.1
(22) Date de dépôt: 26.05.2009
(51) Int. Cl.: A61F 2/00

(54) **Dispositif d'ancrage et de guidage, procédé de fabrication d'un tel dispositif et implant prothétique équipé d'un tel dispositif**
Vorrichtung zur Verankerung und Leitung, Verfahren zur Herstellung einer derartigen Vorrichtung und Implantat mit einer derartigen Vorrichtung
Anchoring and guiding device, manufacturing method and associated implant

(30) Priorité: 29.05.2008 FR 0853506
(43) Date de publication de la demande: 02.12.2009
(73) Titulaire: Cousin Biotech, 59117 Wervicq Sud (FR)
(72) Inventeur: Solecki, Gilles, 59390, Lannoy (FR); Dalle, Valery, 59170, Croix (FR); Kamche, Farid, 59200, Tourcoing (FR)
(74) Mandataire: Cochonneau, Olivier

(56) Documents cités:
- WO-A-03/034891
- WO-A-2006/048885
- WO-A-2006/073582
- US-A1- 2008 021 356

## Description

La présente invention concerne un dispositif d'ancrage et de guidage implantable d'un implant prothétique, notamment pour la cure de l'incontinence urinaire.

La présente invention concerne également un procédé de fabrication d'un dispositif d'ancrage et de guidage implantable, et un implant prothétique équipé d'un dispositif d'ancrage et de guidage implantable, notamment pour l'incontinence urinaire.

L'incontinence urinaire est définie comme toute perte involontaire d'urine dont se plaint le patient. L'incontinence urinaire peut être de trois types : d'effort, par impériosité ou mixte, c'est-à-dire d'effort avec une composante variable d'impériosité.

L'incontinence urinaire d'effort est caractérisée par une fuite involontaire d'urine par le méat urétral, non précédée du besoin d'uriner, qui survient à l'occasion d'un effort tel que la toux, le rire, l'éternuement, le saut, la course, le soulèvement de charges ou toute autre activité physique augmentant la pression intra-abdominale. Elle est due le plus souvent à une hyper mobilité cervico-urétrale lors de l'effort et plus rarement à une insuffisance sphinctérienne.

Parmi les principaux traitements chirurgicaux proposés pour corriger l'incontinence urinaire d'effort, on distingue le traitement par soutènement urétral à l'aide d'une bandelette. Cette bandelette est mise en place sans tension, sous la partie moyenne de l'urètre, et est destinée à supporter l'urètre pendant l'effort, empêchant ainsi toute fuite d'urine. On distingue plusieurs types de bandelettes associées à des voies chirurgicales différentes. On connaît ainsi la bandelette TVT (Tension-Free Vaginal tape) associée au traitement chirurgical par voie rétro-pubienne et la bandelette TOT (Trans-Obturator Tape) associée au traitement chirurgical par voie trans-obturatrice. L'introduction de la voie trans-obturatrice (TOT) en remplacement de la voie rétro-pubienne (TVT) a permis de réduire significativement le taux de complications vésicales, hémorragiques et digestives.

Dans le cas de la voie rétro-pubienne, une incision est pratiquée dans le vagin, sous l'urètre, et deux incisions sont pratiquées dans la peau de l'abdomen. La bandelette TVT est alors placée sous l'urètre à l'aide d'un instrument spécifique puis remonte de part et d'autre de la vessie pour venir s'ancrer dans la paroi abdominale. Cette méthode est délicate à mettre en oeuvre. De plus, il existe un risque de perforation de la vessie lors de la manipulation des aiguilles utilisées pour la pose de la bandelette. Des conséquences graves ont également été constatées telle qu'une perforation de l'artère iliaque ou encore de l'intestin grêle, entraînement des complications viscérales parfois mortelles. C'est la raison pour laquelle il est indispensable, avec cette méthode chirurgicale de pratiquer une cystoscopie.

Dans le cas de la voie trans-obturatrice, des incisions sont réalisées d'une part sur la paroi vaginale et d'autre part sur la face interne de chaque cuisse en regard des trous obturateurs. La bandelette TOT utilisée dans le cadre de cette méthode comporte une partie centrale disposée sous l'urètre et est conçue pour traverser les trous obturateurs et venir ensuite s'ancrer par ses extrémités au niveau de l'aine. Bien que cette méthode présente moins de risques de perforation d'organes importants que la méthode par voie rétro-pubienne, elle ne supprime pas complètement le risque de complications vasculaires en raison de la longueur du trajet occupé par la bandelette dans le corps.

Une dernière technique consiste à utiliser une mini bandelette destinée à être disposée sous l'urètre de manière identique au procédé par la voie trans-obturatrice, à la différence qu'une fois implantée, la mini bandelette ne franchit ni l'espace rétro-pubien, ni le foramen obturateur, ni la membrane obturatrice.

Les bandelettes du type TOT ou TVT comportent des projections latérales et longilignes, de l'ordre de 10 cm à 15 cm, se projetant de la partie centrale de soutènement de l'urètre et destinées à venir s'accrocher en des points distants de ladite partie centrale de sorte que les frottements exercés sur les trajets anatomiques maintiennent la bandelette ancrée dans sa position d'implantation.

La mini bandelette ne comporte pas de telles projections longilignes suivant des trajets anatomiques éloignés de la partie centrale de sorte que des moyens d'ancrage implantables sont disposés sur celle-ci.

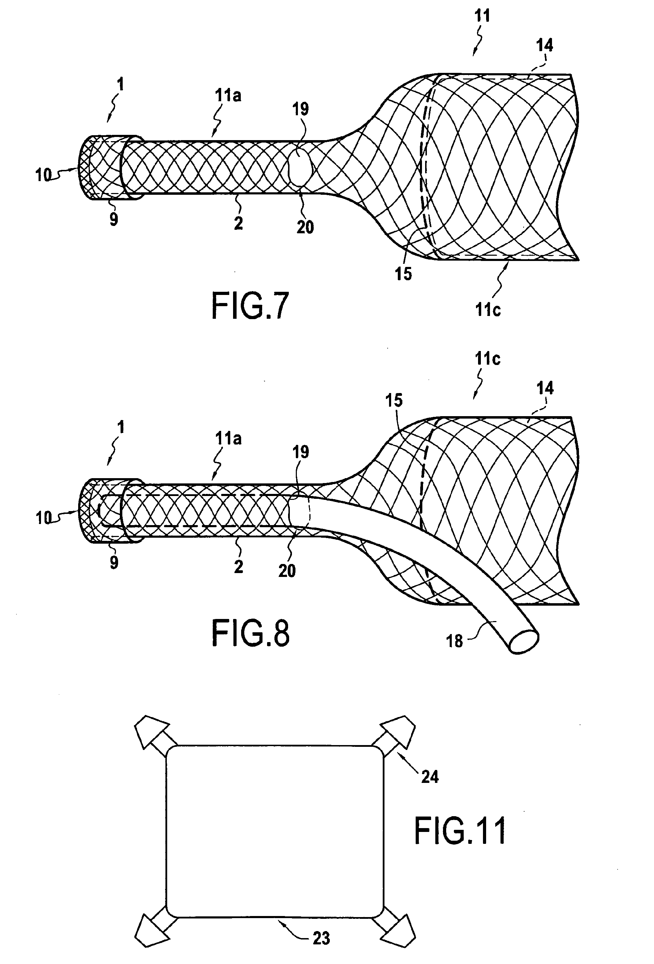

On connaît ainsi dans EP 1.324.705 B1 des mini bandelettes munies à chacune de leurs extrémités d'une ancre de laquelle se projettent de multiples saillies arrangées dans une configuration en arbre de Noël. Lesdites saillies fléchissent dans le sens d'introduction de l'ancre afin de faciliter le passage de celle-ci dans les tissus mous, en particulier dans le tissu mou fibro-graisseux de la zone rétro-pubienne. L'ancrage est assuré par la résistance opposée par les saillies déployées lorsqu'une traction est exercée sur l'ancre dans un sens opposé à celui de son introduction. Ces ancres étant injectées sont rigides et donc blessantes pour les tissus. Il n'est pas possible de les retirer sans disséquer les tissus une fois que les ailettes se sont déployées et sont en appui plan sur une couche de tissu, tel que représenté à la figure 8C.

Lors de l'implantation, quelle que soit la technique utilisée et décrite précédemment, la patiente étant sous anesthésie locale, le praticien dispose dans un premier temps la bandelette dans sa position d'implantation, la partie centrale étant notamment sous l'urètre, et demande à la patiente de tousser, si il observe des fuites urinaires, il doit repositionner la bandelette et donc facilement retrouver les extrémités de celles-ci. Or, une fois implantée, seule une petite portion de la partie centrale de la bandelette est visible pour le praticien. Les moyens d'ancrage connus dans l'état de la technique, et notamment l'ancre de type arbre de Noël décrite ci-dessus, ne comprennent pas de moyens de guidage permettant au praticien de retrouver facilement et de manière sécurisée les extrémités de la bandelette pour ajuster la position de cette dernière.

Le document WO 2006/073582 décrit une bandelette avec un dispositif d'ancrage et de guidage comprenant un élément textile plat et une poche d'ancrage à l'extrémité de l'élement textile plat, avec une partie semi-rigide reliant les bords de l'élement plat et de la poche.

La présente invention a pour objet de proposer un dispositif d'ancrage et de guidage implantable résolvant les inconvénients mentionnés précédemment, permettant de corriger efficacement l'incontinence urinaire d'effort en diminuant les risques de complication opératoire.

Un autre objet est de proposer un dispositif d'ancrage et de guidage implantable simple à utiliser, peu onéreux, facile à fabriquer, et susceptible d'être posé et maintenu dans sa position d'implantation sans tension. Un autre objet est de proposer un dispositif d'ancrage et de guidage moins traumatisant pour les tissus mous que les dispositifs d'ancrage connus et facile à repositionner.

La présente invention a pour objet un dispositif d'ancrage et de guidage implantable d'un implant prothétique, notamment pour l'incontinence urinaire, comprenant:
- un premier élément textile tubulaire, notamment une tresse, dont une première extrémité est solidarisée ou solidarisable audit implant,
- une collerette externe d'ancrage formée à partir d'au moins une couche d'au moins un second élément textile tubulaire et enveloppant la seconde extrémité dudit premier élément tubulaire, et
- une partie distale semi-rigide reliant les parties extrêmes du premier élément tubulaire et de la collerette.

De préférence, le dispositif selon la présente invention est adapté pour l'ancrage et le guidage d'implants dans des tissus mous.

Avantageusement, le dispositif d'ancrage et de guidage étant dans un matériau textile, il favorise la fibrose, laquelle renforce l'ancrage mécanique dudit implant dans sa position d'implantation. De plus, ledit dispositif étant par définition souple, il est moins traumatisant pour les tissus dans lesquels il est introduit que des dispositifs d'ancrage rigides.

La collerette textile souple fait ici office d'ancre mécanique. Le premier élément tubulaire se prolonge à partir de la partie distale semi-rigide à l'intérieur de la collerette.

Les premier et second éléments tubulaires textiles peuvent être tissés, tricotés ou tressés, et comportent de préférence des jours délimités par les fils constituant lesdits éléments. Le tricotage peut s'effectuer sur un métier circulaire. Lesdits éléments peuvent également être formés à partir d'un panneau tricoté ou tissé dont les bords sont soudés, par exemple par ultrasons, ou cousus en sorte de former un tube.

Les premier et second éléments tubulaires comprennent des fils multifilamentaires et/ou des monofilaments, de préférence des monofilaments ayant un diamètre compris dans l'intervalle [0.05 ; 0.50] mm. Les fils multifilamentaires et/ou monofilaments sont de préférence à base d'un ou plusieurs polymères choisis parmi les polymères suivants : polypropylène, polyéthylène, polytéréphtalate d'éthylène, polyamide 6-6 ou 4-6.

De préférence, les premier et second éléments tubulaires textiles sont des tresses. En effet, le demandeur s'est aperçu que les éléments tubulaires tressés offrent dans le cadre du dispositif d'ancrage et de guidage selon la présente invention, une meilleure stabilité dimensionnelle, une bonne mémoire de forme tout en permettant une fabrication économique et simple.

La collerette étant formée à partir d'un second élément tubulaire est creuse ; elle peut donc servir avantageusement de moyen de positionnement en recevant l'extrémité d'un outil chirurgical, notamment d'un ancillaire. La position de la collerette une fois implantée peut ainsi être travaillée sans déplacer le premier élément tubulaire et l'implant prothétique de leurs positions d'implantation.

La collerette a de préférence une forme générale rectangulaire au repos. Lors de son introduction dans les tissus mous, elle a tendance à se déformer permettant une insertion moins traumatique dans les tissus qu'une ancre rigide. Lorsqu'on exerce une traction sur la collerette dans une direction opposée à son sens d'introduction, elle a alors tendance à se comprimer et à raccourcir pour adopter une configuration sensiblement trapézoïdale, la base de la collerette à l'opposé de la partie distale vient alors en appui sur les tissus environnants.

Plus la collerette a tendance à se comprimer et adopter une forme trapézoïdale, plus la résistance au déplacement de la collerette dans les tissus dans une direction opposée au sens de son introduction, est élevée. Le demandeur a ainsi observé que cet effet est amélioré lorsque la collerette est formée à partir d'une tresse. En effet, lorsque l'on pince une tresse au repos en deux zones distinctes puis que l'on rapproche ces deux zones, la tresse se comprime facilement pour former un cylindre.

Lors de l'implantation du dispositif d'ancrage et de guidage dans le tissu mou fibro-graisseux rétro-pubien, la collerette textile, par définition poreuse, emmagasine dans son volume intérieur de la graisse, ce qui améliore sa résistance au déplacement, notamment dans une direction opposée au sens de son introduction et donc corrélativement sa capacité d'ancrage.

Dans une variante, la partie distale est formée par assemblage des parties extrêmes superposées desdits éléments tubulaires. Cet assemblage peut notamment être réalisé par thermofusion lorsque les éléments textiles tubulaires sont constitués de/ou comprennent des fils thermofusibles ; il peut être également réalisé par fusion chimique ou par collage.

Dans une variante, la collerette externe d'ancrage comporte de deux à quatre couches superposées.

Le nombre de couches superposées a un impact d'une part sur la rigidité de la collerette, et donc sur sa capacité à s'ancrer mécaniquement en particulier dans des tissus mous, et d'autre part sur la stabilité dimensionnelle de la collerette.

Dans une variante, tous les éléments textiles tubulaires sont identiques.

Avantageusement, le dispositif d'ancrage et de guidage est formé à partir d'un seul élément tubulaire, ce qui simplifie sa fabrication.

Dans une variante, au moins certains des fils constitutifs du ou des éléments textiles tubulaires de la collerette ont un titrage supérieur à celui des fils constitutifs du premier élément tubulaire.

Cette disposition permet de rigidifier la collerette et donc d'améliorer sa capacité d'ancrage dans les tissus mous. La collerette textile conserve cependant une excellente souplesse lui permettant de se déformer et de ne pas déchirer les tissus lors de l'insertion du dispositif d'ancrage et de guidage dans le corps.

Dans une variante, le premier élément tubulaire comporte vers sa première extrémité une ouverture apte à permettre le passage d'un outil de placement dans ledit premier élément jusqu'à venir en butée intérieurement contre la partie distale.

Dans une variante, le premier élément tubulaire est une tresse et en ce que l'ouverture apte à permettre le passage d'un outil de placement est formée par l'un des jours délimité par les fils constituant ladite tresse.

Dans ce cas, la tresse est de préférence fabriquée en sorte de présenter des jours déformables ; l'outil chirurgical est en effet inséré dans un jour en repoussant les fils adjacents les uns contre les autres. Des outils chirurgicaux variés et standard peuvent ainsi être utilisés.

Dans les deux variantes précédentes, le premier élément tubulaire fait office de gaine recevant l'outil chirurgical et assure ainsi la fonction de moyen de guidage. Le praticien remonte alors l'outil de placement à l'intérieur dudit premier élément tubulaire vers la collerette jusqu'à venir en butée intérieure contre la partie distale. Lors d'une intervention chirurgicale, le praticien peut ainsi facilement repérer l'emplacement de la collerette implantée et ajuster de manière sécurisée la position d'implantation de l'implant prothétique, en particulier s'agissant d'un implant prothétique pour le traitement de l'incontinence urinaire.

L'outil de placement peut être un outil chirurgical tel qu'un ancillaire ou un guide, tel qu'un tube apte à recevoir ledit outil chirurgical. Le tube est dans un matériau à faible coefficient de frottement, par exemple en polypropylène ou en PTFE. Le guide peut également être une tresse.

Dans une variante, la partie distale a une forme annulaire apte à recevoir en butée l'extrémité de l'outil de placement, notamment l'extrémité pointue d'un ancillaire.

Cette disposition permet de renforcer le serrage de l'extrémité de l'outil de placement et donc de faciliter la manipulation et le positionnement de la collerette.

La présente invention a pour objet selon un deuxième aspect un procédé de fabrication d'un dispositif d'ancrage et de guidage implantable d'un implant prothétique selon l'une des variantes décrites ci-dessus. Ledit procédé consiste de manière caractéristique à effectuer les opérations suivantes :
- à partir d'un élément tubulaire constitué de fils thermofusibles dont une première extrémité est solidaire de ou solidarisable audit implant ;
- à replier sur elle-même la seconde extrémité dudit élément tubulaire en sorte d'obtenir sur une longueur L une superposition d'au moins deux couches d'élément tubulaire et
- à thermo souder ensemble les parties extrêmes desdites couches superposées sur une longueur ℓ, inférieure à L, en sorte d'obtenir au moins une collerette externe d'ancrage terminée par une partie distale semi-rigide correspondant aux parties extrêmes thermo soudées.

L'élément tubulaire est de préférence une tresse. De préférence, l'extrémité de l'élément tubulaire est repliée plusieurs fois sur lui-même en sorte d'obtenir plusieurs couches et de préférence au moins trois couches superposées. Le demandeur s'est en effet aperçu que trois couches sont nécessaires pour obtenir une stabilité dimensionnelle et une rigidité suffisantes de la collerette.

Avantageusement, le procédé de fabrication est simple et peu onéreux.

La présente invention a pour objet, selon un troisième aspect, un implant prothétique, notamment textile, comprenant au moins un dispositif d'ancrage et de guidage selon l'une des variantes de réalisation décrites ci-dessus.

Le dispositif d'ancrage et de guidage peut être utilisé de manière générale pour la fixation mécanique et le guidage dans l'ajustement de la position d'implantation d'un implant prothétique, par exemple pour la fixation et le positionnement d'une plaque de réfection pariétale.

Dans une variante, l'implant prothétique comprend une bandelette implantable, notamment pour l'incontinence urinaire, dont les deux extrémités sont équipées d'un dispositif d'ancrage et de guidage selon l'une des variantes de réalisation décrites ci-dessus.

Cette bandelette est de préférence destinée à être disposée sous l'urètre, selon une technique chirurgicale identique au procédé par la voie trans-obturatrice, à la différence qu'une fois implantée, ladite bandelette ne franchit ni l'espace rétro-pubien, ni le foramen obturateur, ni la membrane obturatrice.

Dans une variante, la bandelette implantable est réalisée dans le premier élément textile tubulaire.

Dans une variante, la bandelette implantable comporte une partie centrale sensiblement plane obtenue du fait de l'introduction dans le premier élément tubulaire d'une pièce interne, notamment textile, qui est sensiblement plane et qui est apte à déformer localement la configuration tubulaire dudit premier élément.

Dans une variante, la pièce interne est fixée au premier élément tubulaire.

Dans une variante, la bandelette implantable comporte une partie centrale sensiblement plane obtenue du fait de la fixation entre elles des deux parois résultant de la déformation en largeur et de l'aplatissement du premier élément tubulaire.

Les deux précédentes variantes décrivent des moyens de mise à plat de la partie centrale tubulaire de la bandelette, notamment pour le cas où la partie centrale est destinée à être disposée sous l'urètre lorsque la bandelette est destinée à la cure de l'incontinence urinaire.

Ces moyens de mise à plat évitent l'effet « fil à couper le beurre » de la bandelette lorsque celle-ci est tendue dans sa position d'implantation. De préférence, la bandelette n'est pas élastique

L'invention sera mieux comprise, grâce à la description ci-après, qui se rapporte à des modes de réalisation préférés, donnés à titre d'exemples non limitatifs, et expliqués avec références aux dessins schématiques annexés, dans lesquels :
- les figures 1 à 3 représentent des étapes successives du procédé de fabrication d'un premier exemple de dispositif d'ancrage et de guidage selon la présente invention ;
- la figure 4 représente vue de face un premier exemple de bandelette implantable dont les deux extrémités sont équipés du dispositif d'ancrage et de guidage obtenu par la mise en oeuvre du procédé décrit aux figures 1 à 3 ;

- la figure 5 représente vue de face une collerette de la bandelette représentée à la figure 4 ;
- la figure 6 représente un second exemple de bandelette qui diffère de la bandelette représentée aux figures 4 et 5 en ce qu'elle comporte des moyens de mise à plat de sa partie centrale différents ;
- les figures 7 et 8 représentent un jour dans le premier élément tubulaire du dispositif d'ancrage et de guidage obtenu par la mise en oeuvre du procédé décrit aux figures 1 à 3 permettant l'insertion d'un guide pour un outil chirurgical ;
- la figure 9 représente le fonctionnement du dispositif d'ancrage et de guidage des figures 1 à 3 lors de son introduction dans des tissus mous ;
- la figure 10 représente le fonctionnement du dispositif d'ancrage et de guidage des figures 1 à 3 lors de son retrait dans des tissus mous ;
- la figure 11 représente un troisième exemple d'implant prothétique équipé de plusieurs dispositifs d'ancrage et de guidage selon la présente invention.

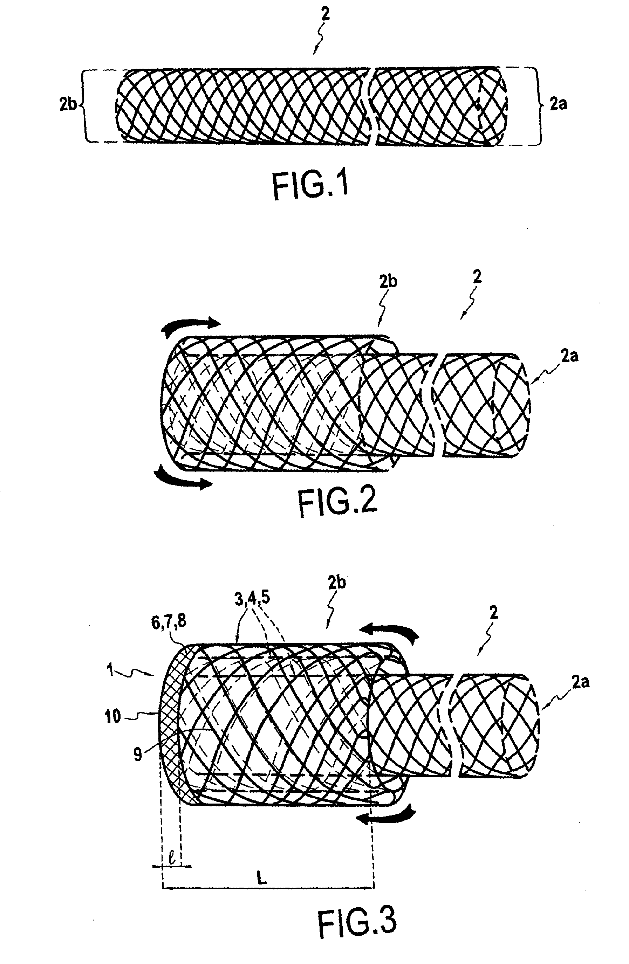

Les figures 1 à 3 illustrent différentes étapes dans la fabrication d'un dispositif d'ancrage et de guidage 1 selon la présente invention obtenu à partir d'un seul élément textile tubulaire 2 faisant office de premier et de second élément tubulaire et présentant une première extrémité 2a et une seconde extrémité 2b. La seconde extrémité 2b de l'élément tubulaire 2 est tout d'abord repliée sur elle-même vers la première extrémité 2a sur une longueur L puis repliée à nouveau sur elle-même dans une direction opposée à ladite première extrémité 2a en sorte d'obtenir la superposition de trois couches 3,4,5 d'élément tubulaire 2. L'élément tubulaire 2 est constitué de fils thermofusibles en sorte de thermosouder facilement ensembles les parties extrêmes 6,7,8 des dites couches 3,4,5 superposées sur une longueur ℓ inférieure à L et former au moins une collerette externe d'ancrage 9 terminée par une partie distale 10 semi-rigide correspondant aux parties extrêmes 6,7,8 thermosoudées. Ainsi, dans cet exemple particulier, les parties extrêmes 6,7,8 sont soudées de telle sorte que la partie distale 10 ait une forme annulaire.

L'assemblage des parties extrêmes pour former la partie distale pourrait être obtenu par d'autres techniques, par exemple par fusion chimique ou par collage.

Le dispositif d'ancrage 1 ainsi obtenu peut être rapporté sur la périphérie d'un implant prothétique.

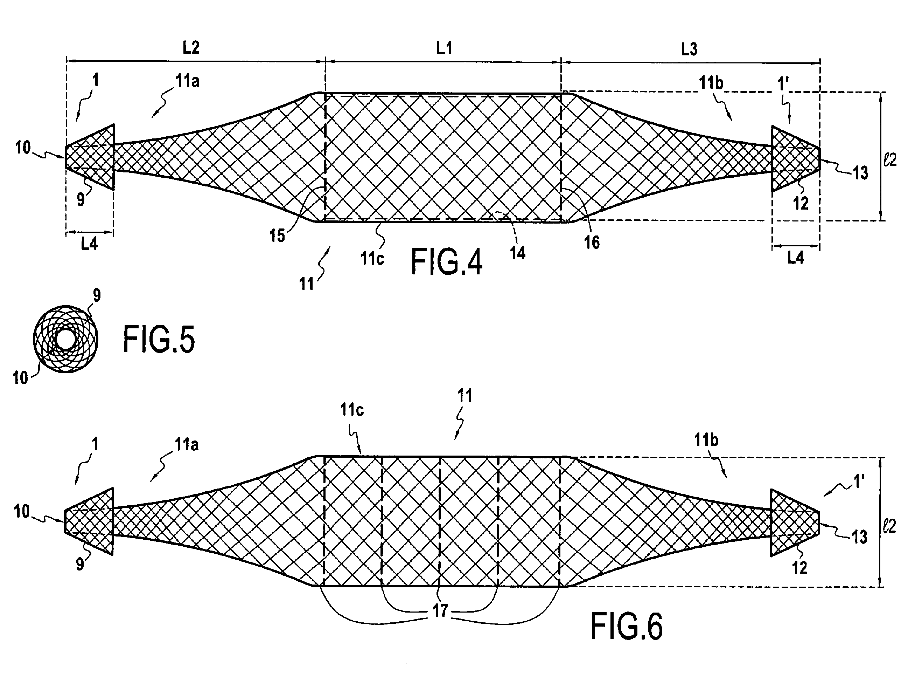

Pour obtenir la bandelette 11 représentée aux figures 4 et 6, on procède avec la première extrémité 2a de l'élément tubulaire 2 telle que pour la seconde extrémité 2b en sorte de former un dispositif d'ancrage et de guidage 1' à chacune des extrémités 11a,11b de la bandelette 11.

La bandelette 11 représentée à la figure 4 est ainsi formée à partir d'un seul élément tubulaire tressé 2. La bandelette 11 comporte deux collerettes 9, formées dans cet exemple précis de deux couches 3,4 d'élément tubulaire 2 superposées. L'élément tubulaire 2 se prolonge de part et d'autre de la partie centrale 11c dans les collerettes 9,12 jusqu'au niveau des parties distales annulaires 10,13.

Afin de maintenir la partie centrale 11c sensiblement plane, une pièce interne 14, dans cet exemple précis un tricot, est introduit à l'intérieur de l'élément tubulaire 2 à travers l'un des jours de celui-ci, puis la pièce interne 14 est fixée selon les coutures 15,16 liant également les deux parois délimitant l'élément tubulaire 2 sur toute la largeur ℓ2 de l'élément tubulaire aplati 2.

La partie centrale 11c peut également être maintenue sensiblement à plat, en fixant les deux parois de l'élément tubulaire 2 entre elles sur toute la largeur ℓ2 de l'élément tubulaire 2 aplati tel que représenté à la figure 6, à l'aide des coutures 17 ou par fusion des fils sur toute la surface de ladite partie centrale 11c.

La fixation peut être réalisée par tous moyens connus de l'état de la technique telle que par couture, soudure thermique, soudure ultrasons ou haute fréquence.

Les dispositifs d'ancrage et de guidage 1,1' sont particulièrement adaptés pour les tissus mous. La bandelette 11 est de préférence utilisée pour le traitement de l'incontinence urinaire et destinée à s'ancrer dans le tissu mou fibro-graisseux de la zone rétro-pubienne ou de la fosse ischio-rectale. Dans cette application, la partie centrale 11c a une longueur L1 de l'ordre de 3 cm, et les projections d'élément tubulaire tressé 2 de part et d'autre de ladite partie centrale 11c ont une longueur L2,L3 de l'ordre de 3 cm à 3,5 cm. L'élément tubulaire 2 est de préférence tressé sur un métier ayant 48 fuseaux à partir de 48 monofilaments dont 24 monofilaments ont un diamètre de l'ordre de 0,13 mm et 24 monofilaments ont un diamètre de l'ordre de 0,20 mm. Les monofilaments sont de préférence choisis parmi l'un des polymères suivants : polypropylène, polyéthylène téréphtalate, polyamide 4-6 ou 6-6, polyéthylène, polymère d'acide lactique ou copolymère d'acide lactique et glycolique.

Les collerettes 9,12 ont une longueur L4 dans cet exemple précis de l'ordre de 6 mm.

L'élément tubulaire tressé 2 comporte des jours 19 délimités par les fils le constituant, lesquels jours 19 sont aptes à se déformer pour l'insertion d'une aiguille, d'un guide et/ou d'un outil chirurgical, tel qu'un ancillaire, par tassement des fils les uns sur l'autre tel que représenté à la figure 7.

Lors de l'implantation des la bandelette 11 pour le traitement de l'incontinence urinaire, un guide 18, par exemple un tube dans un matériau à faible coefficient de frottement, par exemple en polypropylène, est inséré dans un jour 19 de l'élément tubulaire 2, de préférence à partir d'une zone 20 périphérique de la partie centrale 11c. Le guide 18 est inséré jusqu'à venir en butée à l'intérieur de la collerette 9 contre la partie distale 10. Ce tube 18 est gainé par l'élément tubulaire 2 ce qui facilite l'enfilage d'un outil 22 ou d'une aiguille dans ledit tube jusqu'à venir en butée contre la partie distale 10. Ce guide permet de positionner facilement et de manière sécurisée la collerette 9. Une fois la collerette 9 implantée, puis la partie centrale 11c disposée sous l'urètre, le praticien recommence l'opération pour la collerette 12.

Le praticien dispose ainsi de deux moyens distincts de positionnement de la bandelette 11. Le premier moyen de positionnement de la bandelette 11est assuré par chacune des collerettes 9,12. Le praticien enfile ainsi l'extrémité, généralement pointue, de l'outil chirurgical dans une collerette 9,12 jusqu'à ce que ce dernier vienne en butée contre la partie distale annulaire 10,13, ladite extrémité dépassant de la partie distale annulaire 10,13. Ce premier moyen permet de positionner la collerette 9,12 indépendamment de la partie centrale 11c de la bandelette 11. Le second moyen de positionnement est assuré par les portions d'élément tubulaire 2 disposées entre les collerettes 9,12 et la partie centrale 11c, lesquelles font office de gaine du guide et/ou de l'outil chirurgical jusqu'aux parties distales 10,13 desdites collerettes 9,12. Ce second moyen de positionnement fait également office de moyen de guidage puisqu'il permet au praticien de retrouver de manière sécurisée et fiable les collerettes implantées lors d'une intervention chirurgicale.

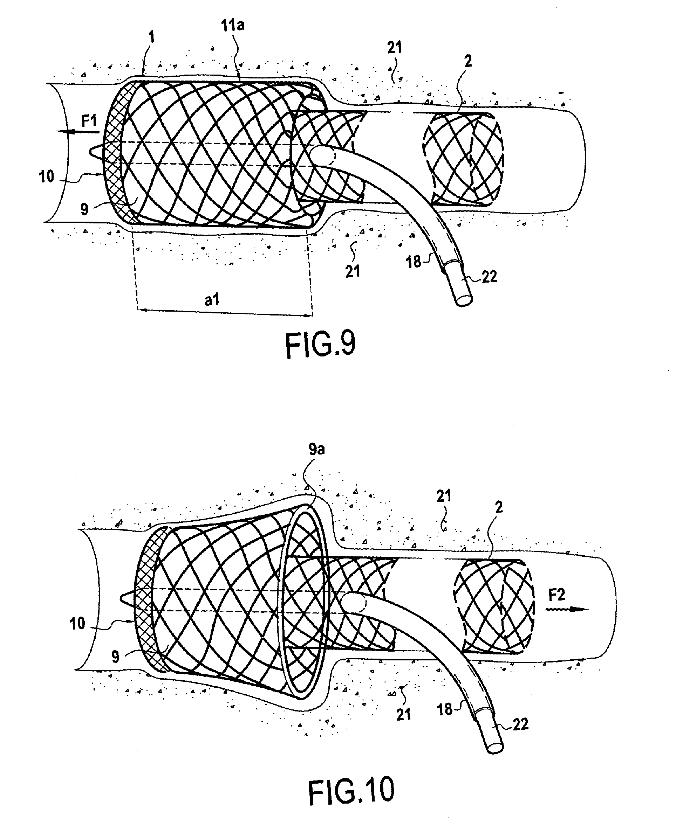

Lors de l'insertion, selon la flèche F1, du dispositif d'ancrage et de guidage 1 dans des tissus mous, en particulier dans les tissus mous fibro-graisseux du rétro-pubien 21, telle que représentée à la figure 9, la collerette 9 étant souple a tendance à se déformer et adopte une forme générale rectangulaire atraumatique pour les tissus 21. Lorsque le praticien exerce une traction sur l'élément tubulaire 2 dans le sens opposé au sens d'introduction du dispositif, selon la flèche F2, la collerette 9 se déploie en sorte d'adopter une configuration générale trapézoïdale dont la base 9a de la collerette 9 vient en appui sur les tissus environnants 21 freinant ainsi sa progression dans la direction, selon F2, opposée à son sens d'introduction. Cependant, la collerette 9 étant souple, cette progression dans une direction opposée au sens d'introduction est moins traumatique qu'une ancre rigide de l'état de la technique. De plus, le praticien peut facilement repositionner la collerette 9 en exerçant à nouveau une traction selon le sens d'introduction F1. La collerette 9 adopte facilement une forme trapézoïdale lorsqu'une traction est exercée sur sa base 9a opposée à la partie distale semi-rigide 10 du fait qu'elle est formée à partir d'une tresse.

Des essais permettant d'évaluer la résistance au déplacement des collerettes 9,12 dans des tissus mous ont été réalisés sur de la viande de porc. Un évidement est tout d'abord pratiqué dans la viande de porc, puis la bandelette 11 est disposée dans celui-ci en appui sur une première collerette 9. La seconde collerette libre 12 est maintenue entre les pinces d'un appareil de mesure, type banc de traction INSTRON®, lequel exerce progressivement une traction sur ladite seconde collerette 12. Il a été observé une résistance au déplacement de l'ordre de 900 grammes pour 10 mm de déplacement avec une bandelette 11 constituée de monofilaments en polypropylène ayant des diamètres de l'ordre de 0,13 mm et de 0,20 mm. Cette résistance est largement suffisante pour obtenir un ancrage mécanique des collerettes 9,12 dans les tissus mous, en particulier dans le tissu mou fibro-graisseux rétro-pubien 21. Cette résistance au déplacement tombe à 500 g dans les mêmes conditions d'essais lorsque la bandelette 11 est constituée de monofilaments de polypropylène ayant tous un diamètre de l'ordre de 0,13 mm.

Enfin, la figure 11 représente un exemple d'implant prothétique 23 comportant quatre dispositifs d'ancrage et de guidage 24. L'implant 23 est dans cet exemple précis un panneau tricoté destiné à la réfection pariétale dans le traitement des hernies. Les dispositifs d'ancrage et de guidage 24 sont disposés à chaque angle de l'implant 23. Le fonctionnement et la fabrication des dispositifs d'ancrage et de guidage 24 sont de préférence identiques aux dispositifs 1,1' décrits précédemment.

## Revendications

1. Dispositif (1) d'ancrage et de guidage implantable d'un implant, notamment pour l'incontinence urinaire, comprenant :
- un premier élément textile tubulaire (2), notamment une tresse, dont une première extrémité (2a) est solidarisée ou solidarisable audit implant,
- une collerette externe d'ancrage (9) formée à partir d'au moins une couche (3,4) d'au moins un second élément textile tubulaire et enveloppant la seconde extrémité (2b) dudit premier élément tubulaire (2), et
- une partie distale (10) semi-rigide reliant les parties extrêmes du premier élément tubulaire (2) et de la collerette (9).

2. Dispositif (1) d'ancrage et de guidage selon la revendication 1 **caractérisé en ce que** la partie distale (10) est formée par assemblage des parties extrêmes (6,7,8) superposées desdits éléments tubulaires, notamment par thermofusion, fusion chimique ou collage.

3. Dispositif (1) d'ancrage et de guidage selon l'une ou l'autre des revendications 1 et 2, **caractérisé en ce que** la collerette externe d'ancrage (9) comporte de deux à quatre couches superposées (3,4).

4. Dispositif (1) d'ancrage et de guidage selon l'une des revendications 1 à 3 **caractérisé en ce que** tous les éléments textiles tubulaires sont identiques (2).

5. Dispositif (1) d'ancrage et de guidage selon l'une des revendications 1 à 3 **caractérisé en ce qu'**au moins certains des fils constitutifs du ou des éléments textiles tubulaires de la collerette (9) ont un titrage supérieur à celui des fils constitutifs du premier élément tubulaire (2).

6. Dispositif (1) d'ancrage selon l'une des revendications 3 à 5 **caractérisé en ce que** deux couches adjacentes (3,4) de la collerette (9) constituent un repli d'un même élément tubulaire (2).

7. Dispositif (1) d'ancrage et de guidage selon l'une des revendications 1 à 6 **caractérisé en ce que** le premier élément tubulaire (2) comporte vers sa première extrémité (2a) une ouverture (19) apte à permettre le passage d'un outil de placement (22) dans ledit premier élément (2) jusqu'à venir en butée intérieurement contre la partie distale (10).

8. Dispositif (1) d'ancrage et de guidage selon la revendication 7, **caractérisé en ce que** le premier élément tubulaire (2) est une tresse et **en ce que** l'ouverture apte à permettre le passage d'un outil de placement (19) est formée par l'un des jours délimité par les fils constituant ladite tresse.

9. Dispositif (1) d'ancrage et de guidage selon l'une des revendications 1 à 8, **caractérisé en ce que** la partie distale (10) a une forme annulaire apte à recevoir en butée l'extrémité de l'organe de placement (22), notamment l'extrémité pointue d'un ancillaire.

10. Procédé de fabrication d'un dispositif d'ancrage et de guidage implantable d'un implant prothétique selon la revendication 2 consistant :
- à partir d'un élément tubulaire (2) dont une première extrémité (2a) est solidaire de ou solidarisable audit implant (11) ;
- à replier sur elle-même la seconde extrémité (2b) dudit élément tubulaire (2) en sorte d'obtenir sur une longueur L une superposition d'au moins deux couches (3,4,5) d'élément tubulaire et ;
- à assembler ensemble, notamment par thermofusion, fusion chimique ou collage, les parties extrêmes (6,7,8) desdites couches superposées (3,4,5) sur une longueur ℓ, inférieure à L, en sorte d'obtenir au moins une collerette (9) externe d'ancrage terminée par une partie distale semi-rigide correspondant aux parties extrêmes assemblées (6,7,8).

11. Implant prothétique, notamment textile, comprenant au moins un dispositif d'ancrage et de guidage (1) selon l'une des revendications 1 à 9.

12. Implant prothétique selon la revendication 11, **caractérisé en ce qu'**il comprend une bandelette (11) implantable, notamment pour l'incontinence urinaire, dont les deux extrémités sont équipées d'un dispositif d'ancrage et de guidage (1).

13. Implant prothétique selon la revendication 12, **caractérisé en ce que** la bandelette (11) implantable est réalisée dans le premier élément textile tubulaire (2).

14. Implant prothétique selon la revendication 13, **caractérisé en ce que** la bandelette (11) implantable comporte une partie centrale (11c) sensiblement plane obtenue du fait de l'introduction dans le premier élément tubulaire (2) d'une pièce interne (14), notamment textile, qui est sensiblement plane et qui est apte à déformer localement la configuration tubulaire dudit premier élément (2).

15. Implant prothétique selon la revendication 14, **caractérisé en ce que** la pièce interne (14) est fixée au premier élément tubulaire.

16. Implant prothétique selon la revendication 13, **caractérisée en ce que** la bandelette (11) implantable comporte une partie centrale (11c) sensiblement plane obtenue du fait de la fixation entre elles des deux parois résultant de la déformation en largeur et de l'aplatissement du premier élément tubulaire (2).

## Claims

1. An implantable anchoring and guide device (1) for anchoring and guiding an implant, in particular for urinary incontinence, said device comprising:
• a first tubular textile element (2), in particular a braid, a first end (2a) of which is secured to or securable to said implant;
• an anchoring outer collar (9) formed from at least one layer (3, 4) of at least one second tubular textile element and enveloping the second end (2b) of said first tubular element (2); and
• a semi-rigid distal portion (10) interconnecting the end portions of the first tubular element (2) and of the collar (9).

2. An anchoring and guide device (1) according to claim 1, **characterized in that** the distal portion (10) is formed by assembling together the superposed end portions (6, 7, 8) of said tubular elements, in particular by heat sealing, chemical fusion, or adhesive bonding.

3. An anchoring and guide device (1) according to claim 1 or claim 2, **characterized in that** the anchoring outer collar (9) is made up of from two to four superposed layers (3, 4).

4. An anchoring and guide device (1) according to any one of claims 1 to 3, **characterized in that** all of the tubular textile elements are identical (2).

5. An anchoring and guide device (1) according to any one of claims 1 to 3, **characterized in that** at least some of the component threads of the tubular textile element(s) of the collar (9) are coarser than the component threads of the first tubular element (2).

6. An anchoring device (1) according to any one of claims 3 to 5, **characterized in that** two adjacent layers (3, 4) of the collar (9) are constituted by the same tubular element (2) that is folded over.

7. An anchoring and guide device (1) according to any one of claims 1 to 6, **characterized in that**, in the vicinity of its first end (2a), the first tubular element (2) is provided with an opening (19) suitable for enabling a placement tool (22) to pass through into said first element (2) until it comes into internal abutment against the distal portion (10).

8. An anchoring and guide device (1) according to claim 7, **characterized in that** the first tubular element (2) is a braid, and **in that** the opening suitable for enabling a placement tool (19) to pass through is formed by one or more gaps defined by the component threads of said braid.

9. An anchoring and guide device (1) according to any one of claims 1 to 8, **characterized in that** the distal portion (10) has an annular shape suitable for receiving in abutment the end of the placement member (22), in particular the pointed end of an ancillary instrument.

10. A method of manufacturing an implantable anchoring and guide device for anchoring and guiding a prosthetic implant according to claim 2, said method consisting in:
• starting from a tubular element (2) having a first end (2a) secured to or securable to said implant (11);
• folding over the second end (2b) of said tubular element (2) so as to obtain, over a length L, superposition of at least two layers (3, 4, 5) of tubular element; and
• assembling together, in particular by heat sealing, by chemical fusion, or by adhesive bonding, the ends (6, 7, 8) of said superposed layers (3, 4, 5) over a length ℓ less than L so as to obtain at least one outer anchoring collar (9) terminated by a semi-rigid distal end corresponding to the assembled-together end portions (6, 7, 8).

11. A prosthetic implant, in particular a textile prosthetic implant, including at least one anchoring and guide device (1) according to any one of claims 1 to 9.

12. A prosthetic implant according to claim 11, **characterized in that** it comprises a piece of implantable tape (11), in particular for urinary incontinence, each end of which is equipped with a respective anchoring and guide device (1).

13. A prosthetic implant according to claim 12, **characterized in that** the implantable tape (11) is formed in the first tubular textile element (2).

14. A prosthetic implant according to claim 13, **characterized in that** the implantable tape (11) has a substantially plane central portion (11c) obtained by inserting an internal piece (14), in particular a textile internal piece, into the first tubular element (2), which piece is substantially plane and is adapted to deform the tubular configuration of said first element (2) locally.

15. A prosthetic implant according to claim 14, **characterized in that** the internal piece (14) is fastened to the first tubular element.

16. A prosthetic implant according to claim 13, **characterized in that** the implantable tape (11) has a substantially plane central portion (11c) that is obtained by fastening together the two walls resulting from widthwise deformation and from flattening of the first tubular element (2).

## Patentansprüche

1. Implantierbare Vorrichtung (1) zum Verankern und Führen eines Implantats, insbesondere für die Harninkontinenz, umfassend:
- ein erstes röhrenförmiges Textilelement (2), insbesondere ein Geflecht, von welchem ein erstes Ende (2a) mit dem Implantat fest verbunden oder fest verbindbar ist,
- einen äußeren Verankerungskragen (9), der aus wenigstens einer Lage (3, 4) wenigstens eines zweiten röhrenförmigen Textilelements gebildet ist und das zweite Ende (2b) des ersten röhrenförmigen Elements (2) umgibt, und
- einen halbstarren distalen Teil (10), der die äußersten Teile des ersten röhrenförmigen Elements (2) und des Kragens (9) verbindet.

2. Verankerungs- und Führungsvorrichtung (1) nach Anspruch 1, **dadurch gekennzeichnet, daß** der distale Teil (10) durch Verbinden der übereinander angeordneten äußersten Teile (6, 7, 8) der röhrenförmigen Elemente, insbesondere durch Thermofusion, chemisches Verschmelzen oder Verkleben gebildet ist.

3. Verankerungs- und Führungsvorrichtung (1) nach dem einen oder anderen der Ansprüche 1 und 2, **dadurch gekennzeichnet, daß** der äußere Verankerungskragen (9) zwei bis vier übereinander angeordnete Lagen (3, 4) umfaßt.

4. Verankerungs- und Führungsvorrichtung (1) nach einem der Ansprüche 1 bis 3, **dadurch gekennzeichnet, daß** alle röhrenförmigen Textilelemente identisch sind (2).

5. Verankerungs- und Führungsvorrichtung (1) nach einem der Ansprüche 1 bis 3, **dadurch gekennzeichnet, daß** wenigstens einige der Fäden, die das oder die röhrenförmige(n) Textilelement(e) des Kragens (9) bilden, eine höhere Feinheitsnummer als die das erste röhrenförmige Element (2) bildenden Fäden haben.

6. Verankerungs- und Führungsvorrichtung (1) nach einem der Ansprüche 3 bis 5, **dadurch gekennzeichnet, daß** zwei benachbarte Lagen (3, 4) des Kragens (9) einen Umschlag eines gleichen röhrenförmigen Elements (2) bilden.

7. Verankerungs- und Führungsvorrichtung (1) nach einem der Ansprüche 1 bis 6, **dadurch gekennzeichnet, daß** das erste röhrenförmige Element (2) in Richtung seines ersten Endes (2a) eine Öffnung (19) aufweist, die geeignet ist, das Durchführen eines Plazierungswerkzeugs (22) in dem ersten Element (2), bis dieses innen an dem distalen Teil (10) in Anschlag gelangt, zu ermöglichen.

8. Verankerungs- und Führungsvorrichtung (1) nach Anspruch 7, **dadurch gekennzeichnet, daß** das erste röhrenförmige Element (2) ein Geflecht ist und daß die Öffnung, welche geeignet ist, das Durchführen eines Plazierungswerkzeugs (22) zu ermöglichen, von einer der Öffnungen gebildet ist, die durch die das Geflecht bildenden Fäden begrenzt ist.

9. Verankerungs- und Führungsvorrichtung (1) nach einem der Ansprüche 1 bis 8, **dadurch gekennzeichnet, daß** der distale Teil (10) eine Ringform aufweist, die geeignet ist, das Ende des Plazierungsorgans (22), insbesondere das spitze Ende eines Hilfsinstruments anschlagend aufzunehmen.

10. Verfahren zur Herstellung einer implantierbaren Vorrichtung zum Verankern und Führen eines prothetischen Implantats nach Anspruch 2, darin bestehend:
- ausgehend von einem röhrenförmigen Element (2), von welchem ein erstes Ende (2a) mit dem Implantat (11) fest verbunden oder fest verbindbar ist,
- das zweite Ende (2b) des röhrenförmigen Elements (2) auf sich selbst umzuschlagen, um über eine Länge L eine Schichtung aus wenigstens zwei Lagen (3, 4, 5) eines röhrenförmigen Elements zu erhalten, und
- die äußersten Teile (6, 7, 8) der übereinander liegenden Lagen (3, 4, 5) über eine Länge 1, die kleiner als die Länge L ist, miteinander zu verbinden, insbesondere durch Thermofusion, chemisches Verschmelzen oder Verkleben, um wenigstens einen äußeren Verankerungskragen (9) zu erhalten, der mit einem halbstarren distalen Teil, welcher den verbundenen äußersten Teilen (6, 7, 8) entspricht, abschließt.

11. Prothetisches Implantat, insbesondere Textilimplantat, das wenigstens eine Verankerungs- und Führungsvorrichtung (1) nach einem der Ansprüche 1 bis 9 umfaßt.

12. Prothetisches Implantat nach Anspruch 11, **dadurch gekennzeichnet, daß** es ein implantierbares Band (11), insbesondere für die Harninkontinenz umfaßt, dessen beide Enden mit einer Verankerungs- und Führungsvorrichtung (1) ausgestattet sind.

13. Prothetisches Implantat nach Anspruch 12, **dadurch gekennzeichnet, daß** das implantierbare Band (11) aus dem ersten röhrenförmigen Textilelement (2) gebildet ist.

14. Prothetisches Implantat nach Anspruch 13, **dadurch gekennzeichnet, daß** das implantierbare Band (11) einen im wesentlichen ebenen mittleren Teil (11c) umfaßt, der dadurch erhalten wird, daß ein Innenteil (14), insbesondere textiles Innenteil, das im wesentlichen eben ist und das geeignet ist, die röhrenförmige Gestalt des ersten Elements (2) lokal zu verformen, in das erste röhrenförmige Element (2) eingeführt wird.

15. Prothetisches Implantat nach Anspruch 14, **dadurch gekennzeichnet, daß** das Innenteil (14) an dem ersten röhrenförmigen Element befestigt ist.

16. Prothetisches Implantat nach Anspruch 13, **dadurch gekennzeichnet, daß** das implantierbare Band (11) einen im wesentlichen ebenen mittleren Teil (11c) umfaßt, der dadurch erhalten wird, daß die beiden Wände, die sich aus der Breitenverformung und aus der Abflachung des ersten röhrenförmigen Elements (2) ergeben, miteinander befestigt werden.
